# EUROPEAN PATENT SPECIFICATION

(11) **EP 2 846 118 B1**
(45) Date of publication and mention of the grant of the patent: **27.02.2019**
(21) Application number: 13884953.4
(22) Date of filing: 01.07.2013
(51) Int. Cl.: F25D 23/10, F25D 29/00, A61L 9/22, F25D 17/04

(54) **FRESH-KEEPING AND STERILIZING APPARATUS FOR REFRIGERATOR, AND CONTROL METHOD**
FRISCHHALTE- UND STERILISIERUNGSVORRICHTUNG FÜR KÜHLSCHRANK UND STEUERUNGSVERFAHREN
APPAREIL DE CONSERVATION AU FRAIS ET DE STÉRILISATION DE RÉFRIGÉRATEUR ET PROCÉDÉ DE COMMANDE

(43) Date of publication of application: 11.03.2015
(73) Proprietor: Hisense Ronshen (Guangdong) Refrigerator Co., Ltd, Ronggui, Shunde Foshan Guangdong 528303 (CN)
(72) Inventor: YANG, Min, Foshan, Guangdong 528303 (CN); KONG, Dong, Foshan, Guangdong 528303 (CN); HU, Zhe, Foshan, Guangdong 528303 (CN); LUO, Ping, Foshan, Guangdong 528303 (CN); LIU, Juncheng, Foshan, Guangdong 528303 (CN)
(74) Representative: Petraz, Gilberto Luigi
(86) International application number: PCT/CN2013/078567
(87) International publication number: WO 2015/000099

(56) References cited:
- WO-A1-2009/008449
- WO-A1-2009/008449
- WO-A1-2012/165114
- WO-A1-2013/065479
- CN-A- 101 091 802
- CN-A- 101 394 067
- CN-A- 103 363 771
- CN-U- 2 045 472
- CN-U- 202 406 944
- CN-U- 203 405 050
- CN-Y- 2 187 765
- JP-A- 2000 300 173
- JP-U- S61 104 181
- US-A1- 2005 268 623

## Description

### TECHNICAL FIELD

The present invention relates to the technical field of refrigeration devices, and more particularly, relates to a preservation and sterilization device for refrigerator and a method for controlling the same.

### BACKGROUND

With the improvement of life, refrigerators have come into thousands of households and have become necessities in household life. People are imposing higher and higher requirements on performance of the refrigerator, and health, sanitation and safety have become eternal subjects during the course of development of the refrigerator.

Refrigerators in the early stage keep food fresh by means of keeping low temperatures therein, but fail to concurrently provide the sterilization and preservation functions. Because bacteria still exists in low temperatures, the food in such refrigerators, especially in refrigeration compartments of these refrigerators, may still decay after being kept for a long period of time, let alone to retain sufficient moisture content and nutrients in the food. Most of the bacteria in the refrigerator are attached on the food. Therefore, a traditional sterilization device fails to reach the bacteria, including the corner far away from the sterilization device. Although the number of bacteria free in a part or in the air is reduced, the bacteria at the corner inside the refrigerator or on the surface of the food breed and produce strange odor.

In recent years, the refrigerator sterilization and preservation technologies are rapidly developing. In addition to the passive sterilization technology of adding an antibacterial material into the inner tank, a lot of active sterilization technologies are available, for example, an ozone generator device, an ion-based sterilization device, an ultraviolet ray-based sterilization device, or the like. The preservation technology for the refrigerator is also gaining development. Prevailing preservation technologies on the current market mainly fall into three categories: simulative induction-based preservation (including photosynthesis preservation and VC induction-based preservation); precise temperature control-based preservation; and sterilization-based preservation, which mainly including silver ion-based sterilization and negative ion-based sterilization technologies.

At present, excellent preservation function and sterilization function are two important performance indicators for refrigerator products. The conventional refrigerator product employs a water cluster separator for the preservation function, which generates a lot of small water clusters favorable to absorption by fruits and vegetables, and thereby achieves better preservation function but poor sterilization function; and employs a positive and negative ion generator for the sterilization function, which generates a large number of positive and negative ions, wherein the positive and negative ions are combined to create a powerful sterilization effect, but the preservation function fails to be achieved.

Document CN 2 045 472 U describes a known electronic freshness-keeping sterilizer for refrigerator. Document JO S61 104181 U and US 2005/268623 A1 describe known refrigerators.

### SUMMARY

The technical problem to be solved in the present invention is to overcome the defect in the prior art, and provide a preservation and sterilization device for refrigerator.

In addition, the present invention further provides a method for controlling the preservation and sterilization device for refrigerator.

To solve the above technical problem, the present invention employs the following technical solution: a preservation and sterilization device for refrigerator, comprising: an electrical control panel, a DC high-voltage power supply, and an ion generator that are sequentially coupled, the ion generator comprising positive and negative ion discharge electrodes; wherein when an input voltage of the ion generator is constant, a DC high voltage output by the ion generator is regulatable to respectively obtain high voltages required for preservation and sterilization.

According to an example, the DC high-voltage power supply comprises a high-voltage trigger module, and a boost module I and a boost module II that are coupled to the high-voltage trigger module, wherein the boost module I and the boost module II achieve voltage boosting by taking coils with different ratios of winding, to respectively obtain the high voltages required for preservation and sterilization.

Specifically, the DC high-voltage power supply is mainly formed of a high-voltage trigger circuit, a selector switch, two boost circuits, and a positive and negative high-voltage filter circuit; when an AC voltage is input to the electrical control panel, the AC voltage is converted to a high frequency AC signal via the high-voltage trigger circuit, and when passing through the selector switch, enters one of the two boost circuits under action of an output electrical signal of the electrical control panel, and a high voltage is thus obtained, wherein when the obtained high voltage passes through the positive and negative high-voltage filter circuit, a positive high voltage and a negative high voltage that are required are obtained.

According to the present invention, the DC high-voltage power supply comprises a high-voltage trigger module I and a high-voltage trigger module II, and a boost module concurrently coupled to the two high-voltage trigger modules, wherein the high-voltage trigger module I and the high-voltage trigger module II employ different current oscillation frequencies, to separately obtain the high voltages required for preservation and sterilization via the boost module.

Specifically, the DC high-voltage power supply is mainly formed of two high-voltage trigger circuits, a selector switch, a boost circuit, and a positive and negative high-voltage filter circuit; after an AC voltage is input to the electrical control panel, when the AC voltage passes through the selector switch, under action of an input electrical signal of the electrical control panel, one of the two high-voltage trigger circuits passes through the boost circuit to obtain a high voltage, wherein when the obtained high voltage passes through the positive and negative high-voltage rectifier and filter circuit, a positive high voltage and a negative high voltage that are required are obtained.

In the preservation and sterilization device for refrigerator, an AC 220 V signal is input to the DC high-voltage power supply or the AC 220 V signal input to the DC high-voltage power supply is replaced by a DC 12 V signal plus an inverter circuit.

In the preservation and sterilization device for refrigerator, the high voltage required for preservation is within a range of 1 KV to 2 KV, and the high voltage required for sterilization is within a range of 2.2 KV to 3 KV.

The preservation and sterilization device according to the present invention may be mounted flexibly. To be specific, the preservation and sterilization device may be mounted at a top portion or a back portion inside the cabinet of the refrigerator or may be embedded at an air duct cover plate of an air-cooled refrigerator.

The present invention further provides a method for controlling the preservation and sterilization device for refrigerator. The method comprises:

S1. enabling, by a user, a preservation function on a function panel, such that a control system controls an ion generator to enter a mode I, whereupon the ion generator generates a stable strong electric field to destroy a cluster structure of a water cluster in the air, and decompose large water clusters in a refrigeration compartment of the refrigerator into small water clusters, thereby facilitating absorption by fruits and vegetables and implementing preservation and moisturizing functions of the refrigeration compartment of the refrigerator; or

S2. enabling, by a user, a sterilization function on a function panel, such that a control system controls an ion generator to enter a mode II, whereupon the ion generator ionizes the air to generate a large number of positive ions and negative ions, wherein the positive ions and the negative ions are combined in the air to give huge energy, thereby causing variation of a bacteria structure and energy transfer in an ambient environment, causing bacteria to die, and implementing the sterilization function of the refrigeration compartment of the refrigerator.

Compared with the prior art, the technical solutions of the present invention achieve the following beneficial effects:

By means of adjusting the design circuit of the DC high-voltage power supply in the ion generator to implement switchover of the output voltage of the ion generator, the present invention solves the technical problem that when the input voltage is constant, the ion generator of a conventional refrigerator only outputs a fixed DC high voltage, which merely singly implements the preservation function or the sterilization function. By means of smart control of the refrigerator, the present invention implements integration of the preservation function and the sterilization function, and achieves the technical effect of concurrently implementing the preservation function and the sterilization function in the refrigeration compartment of the refrigerator by using the same device.

### BRIEF DESCRIPTION OF DRAWINGS

FIG. 1 is a first diagram of electrical principles of an ion-based preservation and sterilization device according to a first example, not part of the invention;
FIG. 2 is a schematic structural diagram of a preservation and sterilization device designed according to the electrical principles embodied in FIG. 1;
FIG. 3 is a second diagram of electrical principles of an ion-based preservation and sterilization device according to the present invention;
FIG. 4 is a schematic structural diagram of a preservation and sterilization device designed according to the electrical principles embodied in FIG. 3;
FIG. 5 is a schematic flowchart of a method for controlling an ion preservation and sterilization device according to the present invention; and
FIG. 6 is a schematic view of applying the ion-based preservation and sterilization device to a refrigerator according to an embodiment of the present invention.

### Reference numerals and denotations thereof

1-preservation and sterilization ion generator device which is mounted at a top portion inside the cabinet of the refrigerator; 2-refrigeration door; 3-door seal strip; 4-freezer door; 5-cabinet; 6-refrigeration layer frame; 7-refrigeration drawer; 8-freezer layer frame; 9-freezer drawer; 10-compressor.

The drawings are for illustration purpose only, but shall not be understood as limitations to the present invention. For better illustration of the following embodiments, some parts or components would be omitted, scaled up or scaled down in the drawings, which are not indicative of the practical sizes. For a person skilled in the art, it shall be understandable that some commonly known structures and description thereof are omitted for brevity.

### DETAILED DESCRIPTION

The present invention is described hereinafter in detail with reference to the attached drawings and specific embodiments.

The present invention discloses a preservation and sterilization device for refrigerator, which comprises: a DC power supply, and an ion generator connected to the DC power supply. When an input voltage of the ion generator is constant, a DC high voltage output by the ion generator is regulatable to respectively obtain high voltages required for preservation and sterilization. The DC high voltages required for implementing the preservation and sterilization functions using the preservation and sterilization device for the refrigerator are subjected to strict requirements. Specifically, the high voltage required for preservation shall be within a range of 1 KV to 2 KV. Because within this voltage range, the decomposition effect of the water clusters is best, if the voltage is lower, and no decomposition effect is present on water clusters; otherwise, higher voltage would destroy the inherited structure of water molecules. When the DC high voltage is lower than 2 KV, the ionization power of the preservation and sterilization device for the refrigerator to an air is weak. The ionization power of the device to the air gradually increases until saturation with the increase of the DC high voltage, and the high voltage of the sterilization is controlled within a range of 2.2 KV to 3 KV under the circumstances of considering electrical safety reliability and sterilization effect of the device. The DC high voltage supplied to the device changes with the material of emitter electrodes of the positive and negative ions and the spacing therebetween.

There are two implementation methods for the preservation and sterilization device according to the present invention:
1. Improving the DC high-voltage power supply of the preservation and sterilization device, adding a selector switch and a boost circuit in the initial circuit, and obtaining, by selecting different boost circuits, the high voltages suitable for decomposing large water clusters and for ionizing the air to generate positive and negative ions.
2. Adding a selector switch and a high-voltage trigger circuit in the initial circuit of the DC high-voltage power supply, obtaining high frequency electrical signal having different frequencies by selecting different high-voltage trigger circuits, and obtaining the high voltages for decomposing large water clusters and for ionizing the air to generate positive and negative ions after the voltage boosting and rectification and filter.

### Example 1

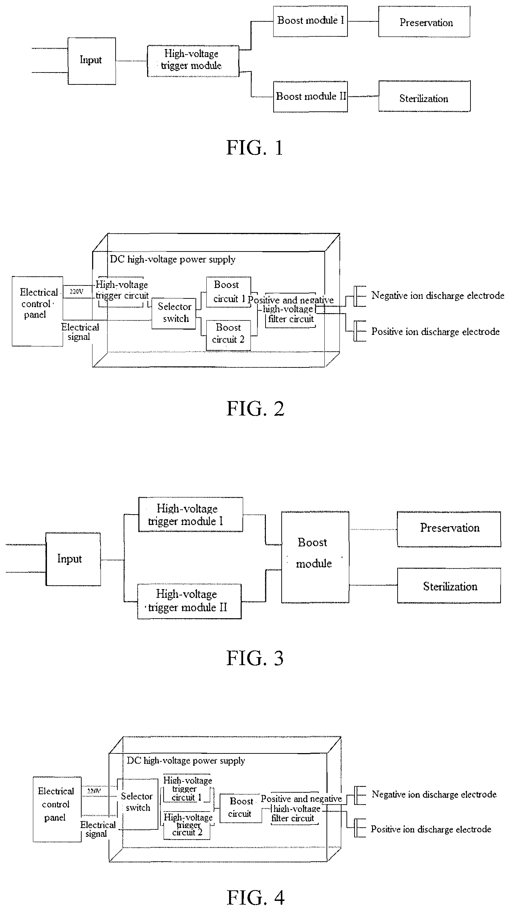

As illustrated in FIG. 1, the DC high-voltage power supply according to example 1 comprises a high-voltage trigger module, and a boost module I and a boost module II that are coupled to the high-voltage trigger module, wherein the boost module I and the boost module II achieve voltage boosting by taking coils with different ratios of winding, to respectively obtain the high voltages required for preservation and sterilization. That is, the boost module I obtains the high voltage required for preservation, and the boost module II obtains the high voltage required for sterilization.

FIG. 2 is a schematic structural diagram of a preservation and sterilization device designed according to the electrical principles embodied in FIG. 1. The preservation and sterilization device according to example 1 is mainly formed of an electrical control panel, a DC high-voltage power supply, and positive and negative ion discharge electrodes. The positive and negative ion discharge electrodes are formed of anti-oxidization metal needles, and the electrical control panel provides an AC voltage and provides an electrical signal to control a selector switch to determine whether DC high-voltage power supply selects a boost loop I or a boost loop II to obtain different output voltages. The DC high-voltage power supply is mainly formed of a high-voltage trigger circuit, a selector switch, two boost circuits, and a positive and negative high-voltage filter circuit.

After an AC voltage is input to the electrical control panel, the AC voltage is converted by the high-voltage trigger circuit to a high frequency AC signal, and enters one of the two boost circuits under action of an output electrical signal of the electrical control panel to obtain a high voltage, wherein when the obtained high voltage passes through the positive and negative high-voltage filter circuit, a positive high voltage and a negative high voltage that are required are obtained. High voltages respectively suitable for decomposing large water clusters and for ionizing the air to generate positive and negative ions are generated when the voltage passes through the two different boost circuits, thereby implementing moisturizing and sterilization functions.

For ease of use under certain circumstances, an AC 220 V signal input to the DC high-voltage power supply of the preservation and sterilization device may be equivalently replaced by a DC 12 V signal plus an inverter circuit.

### Invention embodiment

According to the invention, the DC high-voltage power supply comprises a high-voltage trigger module I and a high-voltage trigger module II, and a boost module concurrently coupled to the two high-voltage trigger modules, wherein the high-voltage trigger module I and the high-voltage trigger module II employ different current oscillation frequencies, to separately obtain the high voltages required for preservation and sterilization via the boost module.

FIG. 4 is a schematic structural diagram of a preservation and sterilization device designed according to the electrical principles embodied in FIG. 3.

The structure of the preservation and sterilization device according to this embodiment is substantially the same as the structure illustrated in example 1 in terms of basic principles. In example 1, different high voltages are obtained by selecting different boost loops, whereas in this embodiment, different high voltages are obtained by selecting different high-voltage trigger circuits. In the structure according to this embodiment, when the AC voltage passes through the selector switch, under action of an input electrical signal of the electrical control panel, one of the two high-voltage trigger circuits passes through the boost circuit to obtain a high voltage; and when the high voltage passes through the positive and negative high-voltage rectifier and filter circuit, a positive high voltage and a negative high voltage that are required are obtained. Since the two high-voltage trigger circuits may generate high frequency voltages having different frequencies, the high voltages subjected to boosting and filter vary, such that high voltages suitable for respectively decomposing large water clusters and for ionizing the air to generate positive and negative ions are obtained, thereby implementing the moisturizing and sterilization functions.

For ease of use under certain circumstances, an AC 220 V signal input to the DC high-voltage power supply of the device may be equivalently replaced by a DC 12 V signal plus an inverter circuit.

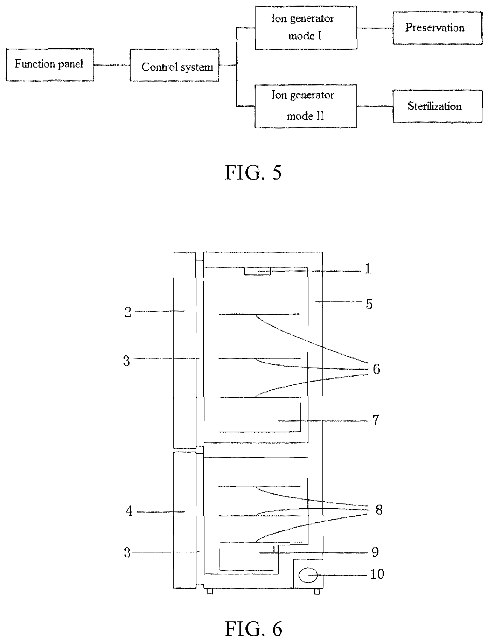

In addition, regardless of whether the preservation and sterilization device according to sample 1 or the invention, the device may be mounted flexibly. To be specific, the preservation and sterilization device may be mounted at a top portion or a back portion inside the cabinet of the refrigerator or may be embedded at an air duct cover plate of an air-cooled refrigerator. FIG. 6 is a schematic view of applying the ion-based preservation and sterilization device to a refrigerator according to the present invention. In the drawing, 1 denotes preservation and sterilization ion generator device which is mounted at a top portion inside the cabinet of the refrigerator; 2 denotes a refrigeration door; 3 denotes a door seal strip; 4 denotes a freezer door; 5 denotes a cabinet; 6 denotes a refrigeration layer frame; 7 denotes a refrigeration drawer; 8 denotes a freezer layer frame; 9 denotes a freezer drawer; and 10 denotes a compressor.

As illustrated in FIG. 5, the present invention further provides a method for controlling a preservation and sterilization device for refrigerator. The method comprises:
1. enabling, by a user, a preservation function on a function panel, such that a control system controls an ion generator to enter a mode I, whereupon the ion generator generates a stable strong electric field to destroy a cluster structure of a water cluster in the air, and decompose large water clusters in a refrigeration compartment of the refrigerator into small water clusters, thereby facilitating absorption by fruits and vegetables and implementing preservation and moisturizing functions of the refrigeration compartment of the refrigerator; or
2. enabling, by a user, a sterilization function on a function panel, such that a control system controls an ion generator to enter a mode II, whereupon the ion generator ionizes the air to generate a large number of positive ions and negative ions, wherein the positive ions and the negative ions are combined in the air to give huge energy, thereby causing variation of a bacteria structure and energy transfer in an ambient environment, causing bacteria to die, and implementing the sterilization function of the refrigeration compartment of the refrigerator.

In conclusion, according to the prior art, the refrigerator employs a water cluster separator for implementing the preservation function, wherein under action of an electric filed force, large water clusters are converted to small water clusters, thereby facilitating absorption by fruits and vegetables; and employs a positive and negative ions generator for implementing the sterilization function, wherein the generated positive and negative ions are combined in the air to give huge energy, thereby causing variation of an inherited bacteria structure and energy transfer, and killing the bacteria. From the perspective of the hardware device, the initial refrigerator employs two different devices for implementing the preservation and sterilization functions, wherein the cost of either of the two devices is the same as that of the other.

The ion generator according to the present invention implements the preservation and sterilization functions on a device by improving the DC power supply. The manufacture cost of the ion generator is merely half of the total cost of the initial water cluster separator and the positive and negative ions generator.

Obviously, the above embodiment is merely an exemplary one for illustrating the present invention, but is not intended to limit the implementation of the present invention. Persons of ordinary skills in the art may derive other modifications and variations based on the above embodiment. All embodiments of the present invention are not exhaustively listed herein. Any modification, equivalent replacement, or improvement made without departing from the principle of the present invention should fall within the protection scope of the present invention.

## Claims

1. A preservation and sterilization device for a refrigerator, comprising: an electrical control panel, a DC high voltage power supply, and an ion generator (1) that are sequentially coupled, the ion generator (1) comprising positive and negative ion discharge electrodes; and in that when an input voltage of the ion generator (1) is constant, a DC high voltage output by the ion generator (1) is regulatable to respectively obtain high voltages required for preservation and sterilization, **characterized in that** the DC high-voltage power supply comprises a high-voltage trigger module I and a high-voltage trigger module II, and a boost module concurrently coupled to the two high-voltage trigger modules, the high-voltage trigger module I and the high-voltage trigger module II employing different current oscillation frequencies, to separately obtain the high voltages required for preservation and sterilization via the boost module.

2. The preservation and sterilization device for refrigerator according to claim 1, wherein the DC high-voltage power supply is mainly formed of two high-voltage trigger circuits, a selector switch, a boost circuit, and a positive and negative high-voltage rectifier and filter circuit; after an AC voltage is input to the electrical control panel, when the AC voltage passes through the selector switch, under action of an input electrical signal of the electrical control panel, one of the two high-voltage trigger circuits passes through the boost circuit to obtain a high voltage, wherein when the obtained high voltage passes through the positive and negative high-voltage rectifier and filter circuit, a positive high voltage and a negative high voltage that are required are obtained.

3. The preservation and sterilization device for refrigerator according to any one of claims 1 or 2, wherein an AC 220 V signal is input to the DC high-voltage power supply or the AC 220 V signal input to the DC high-voltage power supply is replaced by a DC 12 V signal plus an inverter circuit.

4. The preservation and sterilization device for refrigerator according to any one of claims 1 or 2, wherein the high voltage required for preservation is within a range of 1 KV to 2 KV.

5. The preservation and sterilization device for refrigerator according to any one of claims 1 or 2, wherein the high voltage required for sterilization is within a range of 2.2 KV to 3 KV.

6. The preservation and sterilization device for refrigerator according to any one of claims 1 or 2, wherein the preservation and sterilization device is mounted at a top portion or a back portion inside the cabinet (5) of the refrigerator or is embedded at an air duct cover plate of an air-cooled refrigerator.

7. A method for controlling a preservation and sterilization device for a refrigerator according to anyone of the preceding claims 1 to 6, **characterized in that** it comprises:
S1. enabling, by a user, a preservation function on a function panel, such that a control system controls an ion generator (1) to enter a mode I, whereupon the ion generator (1) generates a stable strong electric field to destroy a cluster structure of a water cluster in the air, and decompose large water clusters in a refrigeration compartment of the refrigerator into small water clusters, thereby facilitating absorption by fruits and vegetables and implementing a preservation and moisturizing function of the refrigeration compartment of the refrigerator; or
S2. enabling, by a user, a sterilization function on a function panel, such that a control system controls an ion generator (1) to enter a mode II, whereupon the ion generator (1) ionizes the air to generate a large number of positive ions and negative ions, wherein the positive ions and the negative ions are combined in the air to give huge energy, thereby causing variation of a bacteria structure and energy transfer in an ambient environment, causing bacteria to die, and implementing the sterilization function of the refrigeration compartment of the refrigerator.

## Patentansprüche

1. Haltbarmachungs- und Sterilisierungsvorrichtung für einen Kühlschrank, umfassend: ein elektrisches Steuerpult, eine GS-Hochspannungsstromversorgung und einen Ionengenerator (1), die sequentiell gekoppelt sind, wobei der Ionengenerator (1) positive und negative Ionenabgabeelektroden umfasst; und dass, wenn eine Eingangsspannung des Ionengenerators (1) konstant ist, ein GS-Hochspannungsausgang des Ionengenerators (1) regelbar ist, um jeweils für Haltbarmachung und Sterilisierung erforderliche Hochspannungen zu erhalten, **dadurch gekennzeichnet, dass** die GS-Hochspannungsstromversorgung ein Hochspannungs-Triggermodul I und ein Hochspannungs-Triggermodul II, und ein Boostmodul umfasst, das gleichzeitig mit den zwei Hochspannungs-Triggermodulen gekoppelt ist, wobei das Hochspannungs-Triggermodul I und das Hochspannungs-Triggermodul II unterschiedliche Stromschwingungsfrequenzen verwenden, um die für Haltbarmachung und Sterilisierung erforderlichen Hochspannungen durch das Boostmodul getrennt zu erhalten.

2. Haltbarmachungs- und Sterilisierungsvorrichtung für einen Kühlschrank nach Anspruch 1, worin die GS-Hochspannungsstromversorgung hauptsächlich aus zwei Hochspannungs-Triggerschaltungen, einem Wahlschalter, einer Boostschaltung, und aus einem positiven und negativen Hochspannungsgleichrichter und einer Filterschaltung gebildet ist; nachdem eine WS-Spannung in das elektrische Steuerpult eingegeben ist, wenn die WS-Spannung durch den Wahlschalter, unter Wirkung eines elektrischen Eingabesignals des elektrischen Steuerpultes, läuft, läuft eine der beiden Hochspannungs-Triggerschaltungen durch die Boostschaltung, um eine Hochspannung zu erhalten, worin, wenn die erhaltene Hochspannung durch den positiven und negativen Hochspannungsgleichrichter und die Filterschaltung läuft, eine positive Hochspannung und eine negative Hochspannung, die gefordert sind, erhalten werden.

3. Haltbarmachungs- und Sterilisierungsvorrichtung für einen Kühlschrank nach einem der Ansprüche 1 oder 2, worin ein WS-220V-Signal in die GS-Hochspannungsstromversorgung eingegeben wird oder das WS-220V-Signal, das in die GS-Hochspannungsstromversorgung eingegeben wird, durch ein GS-12V-Signal plus einer Umkehrschaltung ersetzt wird.

4. Haltbarmachungs- und Sterilisierungsvorrichtung für einen Kühlschrank nach einem der Ansprüche 1 oder 2, worin die Hochspannung, die für die Haltbarmachung gefordert ist, in einem Bereich von 1 KV bis 2 KV liegt.

5. Haltbarmachungs- und Sterilisierungsvorrichtung für einen Kühlschrank nach einem der Ansprüche 1 oder 2, worin die Hochspannung, die für die Sterilisierung gefordert ist, in einem Bereich von 2,2 KV bis 3 KV liegt.

6. Haltbarmachungs- und Sterilisierungsvorrichtung für einen Kühlschrank nach einem der Ansprüche 1 oder 2, worin die Haltbarmachungs- und Sterilisierungsvorrichtung an einem oberen Teil oder hinteren Teil innerhalb des Schrankes (5) des Kühlschrankes montiert ist oder an einer Lufteinlassdeckplatte eines luftgekühlten Kühlschrankes eingebettet ist.

7. Verfahren zur Steuerung einer Haltbarmachungs- und Sterilisierungsvorrichtung für einen Kühlschrank nach einem der vorangehenden Ansprüche 1 bis 6, **dadurch gekennzeichnet, dass** es umfasst:
S1. Ermöglichen, durch einen Benutzer, einer Haltbarmachungsfunktion an einem Funktionspult, so dass ein Steuerungssystem einen Ionengenerator (1) ansteuert, um einen Modus I einzugeben, woraufhin der Ionengenerator (1) ein stabiles starkes elektrisches Feld erzeugt, um eine Clusterstruktur eines Wasserclusters in der Luft zu zerstören und um große Wassercluster in einem Kühlungsfach des Kühlschrankes in kleine Wassercluster zu zersetzen, wodurch eine Absorption durch Früchte und Gemüse erleichtert wird und eine Haltbarmachungs- und Befeuchtungsfunktion des Kühlungsfaches des Kühlschrankes durchgeführt werden; oder
S2. Ermöglichen, durch einen Benutzer, einer Sterilisierungsfunktion an einem Funktionspult, so dass ein Steuerungssystem einen Ionengenerator (1) ansteuert, um einen Modus II einzugeben, woraufhin der Ionengenerator (1) die Luft ionisiert, um eine große Anzahl von positiven Ionen und negativen Ionen zu erzeugen, worin die positiven Ionen und die negativen Ionen in der Luft kombiniert werden, um große Energie zu geben, wodurch eine Änderung einer Bakterienstruktur und eine Energieübertragung in einer Außenumgebung verursacht werden, was den Tod der Bakterien verursacht, und die Sterilisierungsfunktion des Kühlungsfaches des Kühlschrankes durchgeführt wird.

## Revendications

1. Dispositif de conservation et de stérilisation pour réfrigérateur comprenant : un tableau de commande électrique, une alimentation électrique haute tension en courant continu et un générateur d'ions (1) qui sont couplés séquentiellement, le générateur d'ions (1) comprenant des électrodes de décharge d'ions positifs et négatifs ; et en ce que lorsqu'une tension d'entrée du générateur d'ions (1) est constante, une haute tension en courant continu délivrée en sortie par le générateur d'ions (1) peut être régulée pour obtenir respectivement des hautes tensions requises pour la conservation et la stérilisation, **caractérisé en ce que** l'alimentation électrique haute tension en courant continue comprend un module de déclenchement haute tension I et un module de déclenchement haute tension II, ainsi qu'un module élévateur couplé simultanément aux deux modules de déclenchement haute tension, le module de déclenchement haute tension I et le module de déclenchement haute tension II utilisant différentes fréquences d'oscillation de courant, pour obtenir séparément les hautes tensions requises pour la conservation et la stérilisation via le module élévateur.

2. Dispositif de conservation et de stérilisation pour réfrigérateur selon la revendication 1, dans lequel l'alimentation électrique haute tension en courant continu est principalement constituée de deux circuits de déclenchement haute tension, d'un sélecteur, d'un circuit élévateur, d'un redresseur à haute tension positive et négative et d'un circuit de filtrage ; après entrée d'une tension en courant alternatif dans le panneau de commande électrique, lorsque la tension en courant alternatif traverse le sélecteur, sous l'action d'un signal électrique d'entrée du panneau de commande électrique, l'un des deux circuits de déclenchement haute tension passe par le circuit élévateur pour obtenir une haute tension, dans lequel lorsque la haute tension obtenue traverse les redresseurs à haute tension positive et négative et le circuit de filtrage, une haute tension positive et une haute tension négative sont obtenues.

3. Dispositif de conservation et de stérilisation pour réfrigérateur selon l'une quelconque des revendications 1 ou 2, dans lequel un signal de courant alternatif de 220 V est entré dans l'alimentation électrique haute tension en courant continu, ou le signal en courant alternatif de 220 V entré dans l'alimentation électrique haute tension en courant continu est remplacé par un signal de courant continu de 12 V plus un circuit inverseur.

4. Dispositif de conservation et de stérilisation pour réfrigérateur selon l'une quelconque des revendications 1 ou 2, dans lequel la haute tension requise pour la conservation est dans la plage de 1 KV à 2 KV.

5. Dispositif de conservation et de stérilisation pour réfrigérateur selon l'une quelconque des revendications 1 ou 2, dans lequel la haute tension requise pour la stérilisation est dans la plage de 2,2 KV à 3 KV.

6. Dispositif de conservation et de stérilisation pour réfrigérateur selon l'une quelconque des revendications 1 ou 2, dans lequel le dispositif de conservation et de stérilisation est monté sur une partie supérieure ou une partie arrière à l'intérieur de l'enceinte (5) du réfrigérateur ou est encastré au niveau d'une plaque de recouvrement de conduit d'air d'un réfrigérateur à refroidissement par air.

7. Procédé de commande d'un dispositif de conservation et de stérilisation pour réfrigérateur selon l'une quelconque des revendications précédentes 1 à 6, **caractérisé en ce qu'**il comprend les étapes consistant à :
S1. activer, par l'intermédiaire d'un utilisateur, une fonction de conservation sur un panneau de fonctions, de sorte qu'un système de commande commande à un générateur d'ions (1) d'entrer dans un mode I, après quoi le générateur d'ions (1) génère un fort champ électrique stable pour détruire une structure en grappe d'une grappe d'eau dans l'air, et décomposer de grandes grappes d'eau situées dans un compartiment de réfrigération du réfrigérateur en petites grappes d'eau, en facilitant ainsi l'absorption par des fruits et des légumes et assurant une fonction de conservation et d'hydratation du compartiment de réfrigération du réfrigérateur ; ou
S2. activer, par l'intermédiaire d'un utilisateur, une fonction de stérilisation sur un panneau de fonctions, de sorte qu'un système de commande commande à un générateur d'ions (1) d'entrer dans un mode II, après quoi le générateur d'ions (1) ionise l'air pour générer un grand nombre d'ions positifs et d'ions négatifs, dans lequel les ions positifs et les ions négatifs sont combinés dans l'air pour donner une importante énergie, en provoquant ainsi une variation d'une structure de bactéries et un transfert d'énergie dans un environnement ambiant, en provoquant la mort des bactéries et en mettant en oeuvre la fonction de stérilisation du compartiment de réfrigération du réfrigérateur.
